(19) Europäisches Patentamt
European Patent Office
Office européen des brevets

(11) EP 1 772 477 A2

(12) # DEMANDE DE BREVET EUROPEEN

(43) Date de publication:
**11.04.2007 Bulletin 2007/15**

(21) Numéro de dépôt: **06119284.5**

(22) Date de dépôt: **22.08.2006**

(51) Int Cl.:
***C08J 3/09*** *(2006.01)* ***C08F 220/00*** *(2006.01)*
***C08F 216/12*** *(2006.01)*

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **06.10.2005 FR 0553032**

(71) Demandeur: **L'Oreal**
**75008 Paris (FR)**

(72) Inventeurs:
- **Mougin, Nathalie**
  **75011, Paris (FR)**
- **Jegou, Gwenaëlle**
  **93190, Livry Gargan (FR)**
- **Giroud, Franck**
  **92110, Clichy (FR)**
- **Samain, Henri**
  **91570, Bievres (FR)**

(74) Mandataire: **Dodin, Catherine**
**L'OREAL**
**DIPI**
**25-29 Quai Aulagnier**
**92665 Asnières-sur-Seine Cedex (FR)**

(54) **Dispersion de particules de polymère, composition la comprenant et procédé de traitement cosmétique**

(57) La présente demande concerne de nouvelles dispersions de particules de polymère stabilisé en surface, dans un milieu non aqueux, dans lesquelles le polymère présente une température de transition vitreuse particulière. L'invention a également pour objet une composition cosmétique ou pharmaceutique comprenant, dans un milieu cosmétiquement ou pharmaceutiquement acceptable, ladite dispersion. L'invention concerne encore un procédé de traitement cosmétique des matières kératiniques, employant ladite composition.

EP 1 772 477 A2

## Description

La présente invention a trait à une nouvelle dispersion de particules de polymères bien particuliers, dispersées dans un milieu non aqueux, ainsi qu'aux compositions notamment cosmétiques ou pharmaceutiques comprenant ladite dispersion.

Parmi les polymères employés dans le domaine cosmétique, et plus particulièrement dans le domaine capillaire, on peut citer les polymères cationiques tels que ceux à base de chlorure de dimethyldiallylammonium, notamment connus pour protéger et/ou embellir le cheveu, grâce à leur forte substantivité. Toutefois, on n'observe aucun effet de mise en forme des cheveux lors de l'emploi de ce type de polymères. Par ailleurs, leur incompatibilité avec la plupart des agents propulseurs ne permet pas de les utiliser dans des produits aérosol tels que les laques.

On connaît par EP1323753, des compositions capillaires comprenant des dispersions aqueuses de polymères hydrophobes cationiques qui apportent des propriétés coiffantes, par exemple lorsqu'ils sont employés dans les shampoings. Toutefois, ces compositions ne présentent pas une très bonne cosmétique en milieu humide, notamment au fur et à mesure des applications des shampooings.

Afin d'obtenir un bon effet coiffant tout en conservant aux compositions des propriétés cosmétiques acceptables, il a été proposé des polymères possédant des propriétés de coiffage, véhiculés dans un solvant cosmétique. Ainsi, dans les documents WO91/15185 et WO98/18433, il est proposé des compositions cosmétiques comprenant des polymères hydrophobes ou non hydrosolubles, véhiculés en solution dans un solvant organique. Toutefois, la nécessité d'employer des polymères solubles dans un milieu organique, implique une faible variabilité des structures chimiques disponibles.

Par ailleurs, on a constaté que les solutions organiques de polymères hydrophobes présentent généralement une viscosité importante, liée à la teneur en polymère de la solution, ce qui rend difficile la formulation ultérieure de ces polymères et de leurs solutions.

Il est par ailleurs connu d'utiliser en cosmétique des dispersions de particules de polymère, généralement de taille nanométrique, dans des milieux organiques, et notamment, par EP749747, des dispersions non aqueuse de particules de polyacrylate ou polyméthacrylate de méthyle, dans une huile de paraffine non volatile ou dans l'isododécane, par exemple. Toutefois, ces dispersions ne permettent pas d'obtenir des propriétés cosmétiques satisfaisantes, notamment en terme d'effet coiffant et de démêlage. Par ailleurs, le toucher des compositions les comprenant peut se révéler peu satisfaisant, notamment quant à la douceur.

La demanderesse a découvert de manière surprenante de nouvelles dispersions de particules de polymères, stabilisées par des stabilisants, dans des milieux non aqueux, qui permettent d'apporter les propriétés de coiffant recherchées, tout en améliorant les propriétés cosmétiques (toucher, douceur, démêlage).

L'invention a donc pour objet une dispersion de particules d'au moins un polymère éthylénique stabilisé en surface par un agent stabilisant, dans un milieu non aqueux constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés;

caractérisée par le fait que ledit polymère éthylénique présente une température de transition vitreuse (Tg) inférieure ou égale à -20°C.

L'invention a également pour objet une composition cosmétique ou pharmaceutique comprenant, dans un milieu cosmétiquement ou pharmaceutiquement acceptable, au moins une dispersion telle que définie ci-dessus.

L'invention permet de préparer des polymères aisément véhiculables, étant donné que les dispersions ont des viscosités peu élevées, ce qui facilite leur mise en oeuvre dans les compositions cosmétiques.

De plus, ces compositions permettent de conférer du volume et de la tenue à la chevelure.

En outre, ces dispersions ou compositions les comprenant apportent des propriétés intéressantes, en particulier en mode rincé. Elles permettent d'obtenir, en plus des effets coiffants et de la tenue, un toucher doux et non collant, une bonne douceur, ainsi qu'une facilité de démêlage du cheveu, en milieu sec et/ou humide.

Ces polymères peuvent être également utilisés dans des produits dits "non rincés" de type laque ou gels/mousses coiffants, et apporter, outre du coiffant, de la cosmétique à la chevelure (toucher, douceur, lissage, démêlage).

Un autre avantage de l'invention réside dans le fait que les particules de polymère peuvent être de très petite taille, notamment nanométrique, ce qui n'est pas le cas avec, par exemple, d'autres types de particules telles que des microsphères dont le diamètre est généralement supérieur à 1 micron. Or, une taille importante de l'ordre du micron a pour inconvénient d'entraîner une certaine visibilité des particules à l'oeil, lorsqu'elles sont dans une composition et lorsqu'elles sont appliquées sur la peau, ainsi qu'une mauvaise stabilité de la composition, notamment dans le temps. Ainsi, les dispersions selon l'invention permettent l'obtention de compositions stables, qui peuvent en outre être transparentes, translucides ou opaques, au choix, selon la taille des particules de polymère qui y sont dispersées.

Les dispersions selon l'invention sont donc constituées de particules, généralement sphériques, d'au moins un polymère éthylénique, stabilisé en surface par un stabilisant, dans un milieu non aqueux.

Les dispersions selon l'invention peuvent notamment se présenter sous forme de nanoparticules de polymères en dispersion stable dans un milieu non aqueux. Les nanoparticules sont de préférence d'une taille comprise entre 5 et

600 nm, notamment 10 à 500 nm, encore mieux 15 à 450 nm, étant donné qu'au-delà d'environ 600 nm, les dispersions de particules deviennent beaucoup moins stables.
Notamment, ces particules restent à l'état de particules élémentaires, sans former d'agglomérats, lorsqu'elles sont en dispersion dans lesdits milieux non aqueux.

Par polymère "éthylénique", on entend un polymère obtenu par polymérisation d'au moins 2 monomères, identiques ou différents, comprenant une insaturation éthylénique.
Ledit polymère éthylénique peut être choisi par l'homme du métier en fonction de ses propriétés, selon l'application ultérieure souhaitée pour la composition. Ces polymères peuvent être en particulier réticulés.

Toutefois, ledit polymère éthylénique présente au moins une température de transition vitreuse (Tg) inférieure ou égale à -20°C, de préférence comprise entre - 150°C et -20°C, notamment entre -100°C et -25°C, préférentiellement entre -95°C et -30°C, voire entre -80°C et -35°C, et encore mieux entre -70°C et -40°C.
De préférence, le polymère ne présente qu'une température de transition vitreuse.
Toutefois, il peut présenter plusieurs températures de transition vitreuse, notamment deux Tg; dans ce cas, la Tg la plus basse doit être inférieure à -20°C.

Dans la présente invention, les Tg (ou température de transition vitreuse) indiquées sont des Tg théoriques déterminées à partir des Tg théoriques des monomères constitutifs du polymère, que l'on peut trouver dans un manuel de référence, tel que le Polymer Handbook, 3ème ed, 1989, John Wiley, selon la relation suivante, dite Loi de Fox :

$$\frac{1}{Tg} = \sum_i \left(\frac{\varpi i}{Tgi}\right)$$

wi étant la fraction massique du monomère i dans le polymère et Tgi étant la température de transition vitreuse de l'homopolymère du monomère i, exprimée en Kelvin (K).

Dans la présente description, on désigne par "monomère de Tg", le monomère dont l'homopolymère a une telle température de transition vitreuse.

Les polymères selon l'invention peuvent être des homopolymères ou des copolymères linéaires, branchés, ou même en étoiles. Ils peuvent être statistiques ou alternés. De préférence, ce sont des copolymères statistiques linéaires.

D'une manière générale, les monomères de Tg inférieure ou égale à -20°C, peuvent représenter 50 à 100% en poids du poids total des monomères initiaux.

Dans un premier mode de réalisation de l'invention, les polymères présents dans la dispersion peuvent être issus de la polymérisation d'un ou plusieurs monomères de Tg inférieure ou égale à -20°C, de préférence comprise entre -150°C et -20°C, notamment entre -100°C et -25°C, préférentiellement entre -95°C et -30°C, voire entre -80°C et -40°C, et encore -70°C et -45°C.
Dans ce mode de réalisation, le monomère de Tg inférieure ou égale à -20°C, ou leur mélange, représente 100% en poids du poids total de monomères initiaux.

Dans un second mode de réalisation de l'invention, les polymères présents dans la dispersion peuvent être issus de la polymérisation d'un ou plusieurs monomères de Tg inférieure ou égale à -20°C, et d'un ou plusieurs monomères dits additionnels de Tg supérieure à -20°C, mais présents en une quantité telle que la Tg globale du polymère est inférieure ou égale à -20°C.
Par exemple, on peut combiner dans le polymère final, un monomère de Tg de l'ordre de 100°C, qui peut être présent à raison de 10-20% en poids du poids total de monomères, et un monomère de Tg de l'ordre de -50°C, qui peut être présent à raison de 80-90% en poids, de manière à obtenir un polymère ayant une Tg d'environ -40°C à -30°C.
Dans ce mode de réalisation, de préférence le monomère additionnel, ou le mélange de tels monomères, peut être présent à raison de 0,01 à 50% en poids, par rapport au poids total des monomères, notamment de 0,1 à 40% en poids, voire de 1 à 30% en poids, ou encore de 5 à 15% en poids. Le monomère de Tg inférieure ou égale à -20°C, ou le mélange de tels monomères, peut alors être présent à raison de 50 à 99,99% en poids, notamment de 60 à 99,9% en poids, voire de 70 à 99% en poids, ou encore de 85 à 95% en poids, par rapport au poids total de monomères.
L'homme du métier saura, sur la base de la loi de Fox et de ses connaissances générales, déterminer les quantités maximales de monomère additionnel susceptible d'être présent dans le polymère de la dispersion, de manière à toujours obtenir au final une dispersion de polymère ayant une Tg inférieure ou égale à -20°C.

Par ailleurs, on a constaté que lorsque la dispersion de polymère selon l'invention comprend un ou plusieurs monomères dits hydrophobes tels que définis ci-après, elle présente de bonnes propriétés de rémanence du dépôt, ainsi que de douceur, et une bonne compatibilité avec les compositions de type après-shampooing.

Ainsi, de préférence, le polymère éthylénique selon l'invention comprend 40% à 100% en poids, par rapport au poids total de monomères, notamment de 60 à 99% en poids, voire de 70 à 98% poids, et encore mieux de 60 à 95% en poids de monomère hydrophobe, seul ou en mélange.

Ce ou ces monomères hydrophobes pourront être choisis parmi les monomères ayant une Tg inférieure ou égale à -20°C et/ou parmi les monomères ayant une Tg supérieure à -20°C.
De préférence, le ou les monomères hydrophobes ont une Tg inférieure à -20°C.

Par "monomère hydrophobe", on entend au sens de la présente invention un monomère ayant une valeur du logarithme du coefficient de partage apparent octanol-1/eau, aussi appelé log P, supérieure ou égale à 2, par exemple comprise entre 2 et 11, de préférence comprise entre 2,5 et 10, notamment entre 3 et 8, voire entre 3,5 et 5.

Les valeurs de log P sont connues et sont déterminées selon un test standard qui détermine la concentration du monomère dans l'octanol-1 et l'eau.
Les valeurs peuvent notamment être calculées à l'aide du logiciel ACD (Advanced Chemistry Development) Software solaris V4.67; elles peuvent également être obtenues à partir de Exploring QSAR : hydrophobic, electronic and steric constants (ACS professional reference book, 1995). Il existe encore un site Internet qui fournit des valeurs estimées (adresse : http://esc.syrres.com/interkow/kowdemo.htm).
Nous indiquons ci-après la valeur du log P de certains monomères usuels, déterminée à l'aide du logiciel ACD :

| | acrylate (* acrylamide) | méthacrylate (* méthacrylamide) |
|---|---|---|
| méthyl (méth)acrylate | 0.793 +/- 0.223 | 1.346 +/- 0.250 |
| éthyl (méth)acrylate | 1.325 +/- 0.223 | 1.877 +/- 0.250 |
| propyl (méth)acrylate | 1.856 +/- 0.223 | 2.408 +/- 0.250 |
| isopropyl (méth)acrylate | 1.672 +/- 0.228 | 2.224 +/- 0.254 |
| n-butyl (méth)acrylate | 2.387 +/- 0.223 | 2.940 +/- 0.250 |
| isobutyl (méth)acrylate | 2.208+/-0.228 | 2.756 +/- 0.254 |
| terbutyl(méth)acrylate | 2.022 +/- 0.238 | 2.574 +/- 0.261 |
| cyclohexyle (méth)acrylate | 2.853+/- 0.226 | 3.405 +/-0.252 |
| octyl(méth)acrylate | 4.513 ± 0.224 | 5.065 +/- 0.521 |
| lauryl(méth)acrylate | 6.638 ± 0.224 | 7.190 +/- 0.251 |
| tridecyl(méth)acrylate | 7.170 ± 0.224 | 7.712 ± 0.251 |
| cétyl (méth)acrylate | 8.764 ± 0.224 | 9.316 ± 0.251 |
| palmityl(méth)acrylate | >9 | >9 |
| stéaryl (méth)acrylate | 9.826 ± 0.224 | 10.379 ± 0.251 |
| behenyl (méth)acrylate | 12.504 ± 0.251 | 11.952 ± 0.225 |
| oléyl (méth)acrylate | 9.308 ± 0.232 | >9 |
| tétrahydrofurfuryl (méth)acrylate | 0,800 ± 0,263 | 1,352 ± 0,283 |
| 2-éthyl hexyl (méth)acrylate | 4,329 ± 0,229 | 4,881 ± 0,254 |
| 2-hydroxyéthyl (méth)acrylate | 0,166 ± 0,258 | 0,718 ± 0,277 |
| éthoxyéthyle (méth)acrylate | 1,335 ± 0,268 | 1,887 ± 0,293 |
| hydroxypropyl (méth)acrylate | 0,383 ± 0,241 | |
| diméthylaminoéthyle (méth)acrylate (MADAME) | | 0,97 |
| N-isopropyle (méth)acrylamide * | 0,195 ± 0,256 | 0,748 ± 0,276 |
| N-octyl (méth)acrylamide * | 3,036 ± 0,253 | 3,558 ± 0,273 |
| N-terbutyl (méth)acrylamide * | 1,02 | |
| N,N-diméthyl (méth)acrylamide* | -0,168 ± 0,556 | 0,906 ± 0,553 |
| N,N-dibutyl (méth)acrylamide* | 3,021 ± 0,557 | 3,573 ± 0,570 |
| acide (méth)acrylique | 0,35 | 0,83 |

Parmi les monomères de Tg inférieure ou égale à -20°C, susceptibles d'être employés pour former la dispersion selon l'invention, on peut citer :

- (i) les esters de l'acide acrylique de formule $CH_2$=CHCOOR1 avec R1 représentant (a) une chaîne carbonée, notamment hydrocarbonée (alkyle), ayant 2 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F), à l'exclusion de la chaîne tertiobutyle; ou bien R1 représentant (b) un groupement polyoxyéthylène comprenant de 5 à 30 motifs d'oxyde d'éthylène; ou encore R1 représentant (c) un groupe -R-($OC_2H_4$)n-H, avec R = alkyle en C1-C12 et n est un entier compris entre 5 et 30 inclus.
  On peut ainsi citer les acrylates d'éthyle, de propyle, de n-butyle, d'isobutyle, de 2-éthylhexyle, d'octyle, d'isooctyle, d'isodécyle, de décyle, de lauryle, de tridécyle, d'hydroxyéthyle et d'hydroxypropyle.

- (ii) les esters de l'acide méthacrylique de formule $CH_2$=C($CH_3$)COOR2 avec R2 représentant (a) une chaîne carbonée, notamment hydrocarbonée (alkyle), ayant 8 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi - OH et les atomes d'halogène (Cl, Br, I et F) ; ou bien R2 représentant (b) un groupement polyoxyéthylène comprenant de 5 à 30 motifs d'oxyde d'éthylène; ou encore R2 représentant (c) un groupe -R-($OC_2H_4$)n-H, avec R = alkyle en C1-C30 et n est un entier compris entre 5 et 30 inclus.
  On peut ainsi citer les méthacrylates d'octyle, d'isooctyle, de décyle, d'isodécyle, de dodécyle, de lauryle, de tridécyle, de myristyle, de cétyle, de palmityle, de stéaryle, de béhényle, d'oléyle.

- (iii) les esters de vinyle de formule $CH_2$=CH-OCO-R3 avec R3 représentant une chaîne carbonée, notamment hydrocarbonée, ayant 2 à 12 atomes de carbone, linéaire ou ramifiée, saturée ou insaturée, parmi lesquels on peut citer le butyrate (ou butanoate) de vinyle, l'éthylhexanoate de vinyle, le néononanoate de vinyle et le néododécanoate de vinyle;

- (iv) les éthers de vinyle de formule $CH_2$=CHOR4 avec R4 représentant une chaîne carbonée, notamment hydrocarbonée, ayant 1 à 12 atomes de carbone, linéaire ou ramifiée, saturée ou insaturée; parmi lesquels on peut citer l'éther de vinyle, le méthylvinyléther, l'éthylvinyléther, l'éthylhexylvinyléther et le butylvinyléther;

- (v) les N-alkyl (méth)acrylamides de formule $CH_2$=CHCONR5R'5 ou $CH_2$=C($CH_3$)CONR5R'5 avec R5 et R'5 représentant indépendamment l'un de l'autre, un atome d'hydrogène ou une chaîne carbonée, notamment hydrocarbonée, ayant 6 à 28 atomes de carbone, linéaire, cyclique ou ramifiée, saturée ou insaturée, éventuellement aromatique (aryle, aralkyle ou alkylaryle), comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F) ; étant donné que l'un au moins des radicaux R5, R'5 est différent de l'hydrogène;
  parmi lesquels on peut citer le N-octylacrylamide et le N-octadécylacrylamide.

Les monomères de Tg inférieure ou égale à -20°C plus particulièrement préférés sont l'acrylate de n-butyle, l'acrylate d'éthylhexyle, l'acrylate d'isobutyle, l'acrylate d'isooctyle et leur mélange.

Parmi les monomères ayant une Tg inférieure ou égale à -20°C et étant de plus hydrophobes, on peut citer les acrylates de n-butyle, d'isobutyle, de 2-éthylhexyle, d'octyle, d'isooctyle, d'isodécyle, de décyle, de lauryle; ainsi que les méthacrylates d'octyle, d'isooctyle, de décyle, d'isodécyle, de dodécyle, de lauryle, de tridécyle, de cétyle, de palmityle, de stéaryle, d'oléyle.

Ainsi qu'il est mentionné plus haut, le polymère en dispersion selon l'invention peut comprendre un ou plusieurs monomères additionnels, de Tg supérieure à - 20°C, de préférence supérieure à 0°C, sous réserve que ce ou ces monomères additionnels, et/ou leur quantité, soient choisis de façon à ce que la Tg globale du polymère soit inférieure ou égale à -20°C.

Ces monomères additionnels peuvent être choisis parmi les monomères suivants :

- (i) les composés vinyliques de formule $CH_2$=CHR6 dans laquelle R6 est

  - un groupe hydroxyle;
  - un groupe alkyle linéaire ou ramifié, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes

d'halogène (Cl, Br, I et F);

- un groupe cycloalkyle en $C_3$ à $C_8$ tel que cyclohexane,
- un groupe aryle en $C_6$ à $C_{20}$ tel que phényle,
- un groupe aralkyle en $C_7$ à $C_{30}$ (groupe alkyle en $C_1$ à $C_4$) tel que 2-phényléthyle ou benzyle,
- un groupe hétérocyclique de 4 à 12 chaînons contenant un ou plusieurs hétéroatomes choisis parmi O, N, et S,
- un groupe hétérocycloalkyle (alkyle de 1 à 4 carbones), tel que furfuryle, furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi -OH, les atomes d'halogène (Cl, Br, I et F).
Des exemples de monomères vinyliques sont le vinylcyclohexane, le styrène et l'acétate de vinyle.

- (ii) les acrylates de formule $CH_2$=CHCOOR7 dans laquelle R7 est un groupe tertiobutyle, un groupe cycloalkyle en C3 à C8; un groupe aryle en C6 à C20 ; un groupe aralkyle en C7 à C30 (groupe alkyle en C1 à C4); un groupe hétérocyclique de 4 à 12 chaînons contenant un ou plusieurs hétéroatomes choisis parmi O, N et S; un groupe hétérocycloalkyle (alkyl de C1 à C4) tel qu'un groupe furfuryle; lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi le groupe hydroxyle et les atomes d'halogène (Cl, Br, I et F).
Des exemples de tels monomères sont les acrylates de tertio-butyle, de t-butylcyclohexyle, de t-butylbenzyle, de furfuryle et d'isobornyle.

- (iii) les méthacrylates de formule $CH_2$=C($CH_3$)COOR8 dans laquelle R8 est :

  - un groupe carboné, notamment hydrocarboné (alkyle) ayant 1 à 6 atomes de carbone, linéaire ou ramifié, tel qu'un groupe méthyle, éthyle, propyle ou isobutyle, ledit groupe alkyle pouvant en outre être éventuellement substitué par un ou plusieurs substituants choisis parmi OH et les atomes d'halogène (Cl, Br, I et F) ;
  - un groupe cycloalkyle en C3 à C8 ;
  - un groupe aryle en C6 à C20 ;
  - un groupe aralkyle en C7 à C30 (groupe alkyle en C1 à C4) ;
  - un groupe hétérocyclique de 4 à 12 chaînons contenant un ou plusieurs hétéroatomes choisis parmi O, N et S;
  - un groupe hétérocycloalkyle (alkyle en C1-C4), tel qu'un groupe furfuryle ;

lesdits groupes cycloalkyle, aryle, aralkyle, ou hétérocyclique ou hétérocycloalkyle pouvant être éventuellement substitué par un ou plusieurs substituants choisis parmi OH, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).
Des exemples de tels monomères sont les méthacrylates de méthyle, d'éthyle, de propyle, de n-butyle, d'isobutyle, de t-butylcyclohexyle, de t-butylbenzyle, de méthoxyéthyle, de méthoxypropyle et d'isobornyle.

- (iv) les (méth)acrylamides de formule $CH_2$=CHCONR9R'9 ou $CH_2$=C($CH_3$)CONR9R'9 dans lesquelles R9 et R'9, identiques ou différents, représentent un atome d'hydrogène ou un groupe alkyle en C1-C5, linéaire ou ramifié, tel qu'un groupe n-butyle, t-butyle, isopropyle.
Des exemples de tels monomères sont le N-butyl(méth)acrylamide, le N-isopropyl(méth)acrylamide, le N,N-diméthyl (méth)acrylamide et le N,N-dibutyl(méth)acrylamide.

Les monomères de Tg supérieure à -20°C plus particulièrement préférés sont les acrylates de furfuryle, d'isobornyle, de tertiobutyle, de tertiobutylcyclohexyle, de tertiobutylbenzyle; les méthacrylates de méthyle, de n-butyle, d'éthyle, d'isobutyle, le styrène, l'acétate de vinyle et le vinylcyclohexane, et leurs mélanges.

Parmi les monomères ayant une Tg supérieure à -20°C et étant de plus hydrophobes, on peut citer les acrylates d'isobornyle, de tertiobutyle, de tertiobutylcyclohexyle, de tertiobutylbenzyle; les méthacrylates de n-butyle, d'isobutyle, le styrène.

Le polymère présent dans la dispersion selon l'invention peut, bien évidemment, comprendre des monomères hydrophiles (c'est-à-dire non hydrophobes ou encore ayant un log P inférieur à 2), qui peuvent avoir une Tg inférieure ou égale à -20°C ou une Tg supérieure à -20°C, ou un mélange de tels monomères hydrophiles.

Parmi les monomères hydrophiles susceptibles d'être employés, on peut citer l'acide acrylique, l'acide méthacrylique, le methacrylate de dimethylaminoethyle (MADAME), le méthacrylamide de diméthylaminopropyle (DMAPMA), la vinylpyridine et la vinylimidazole, le MADQUAT (ou [2-(methacryloyloxy)ethyl] trimethylammonium chloride), le MAPTAC (ou methacrylamidopropyltrimethylammonium chloride), le 2-hydroxyethylmethacrylate, le 2-hydroxyethylacrylate, le N-terbutylacrylamide, le tetrahydrofurfurylmethacrylate, le méthacrylate de méthyle, le méthacrylate d'éthyle, l'acrylate de méthyle, l'acrylate d'éthyle, la vinylpyrrolidone, le vinylacétate, les acrylates ou méthacrylates de formule CH2=C(H, $CH_3$)COOR avec R alkyle pouvant contenir des groupements (OC2H4)m-OR", avec m = 5 à 150 et R" = H ou alkyle de C1 à C30.

Lorsqu'il comporte des fonction acides, le polymère selon l'invention peut être neutralisé par une base organique, par exemple une amine primaire, secondaire ou tertiaire, l'amine pouvant comporter ou non des substituants (hydroxyle), comme l'amino-2-méthyl-2-propanol, et les formes salifiées ou quaternisées de ceux-ci.
Lorsqu'il comporte des fonctions basiques, le polymère selon l'invention peut être neutralisé par des acides organiques qui peuvent comporter un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique, l'acide alpha-hydroxyéthanoïque, l'acide alpha-hydroxyoctanoïque, l'acide alpha-hydroxycaprylique, l'acide ascorbique, l'acide benzoïque, l'acide béhénique, l'acide caprique, l'acide caproïque, l'acide caprylique, l'acide citrique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléïque, l'acide palmitique, l'acide propionique, l'acide sébacique, l'acide stéarique, l'acide tartrique, l'acide téréphtalique, l'acide trimésique, l'acide undécylénique, la propyle-bétaïne, la cocoamidopropylbétaïne, et leurs mélanges.

Les polymères utilisables dans le cadre de la présente invention ont de préférence un poids moléculaire moyen en nombre (Mn) compris entre 2000 à 1 000 0000, notamment entre 3000 et 800 000, et encore mieux entre 4000 et 500 000.

La dispersion de particules de polymères selon l'invention comprend donc un milieu non aqueux, dans lequel sont dispersées lesdites particules.

Ce milieu non aqueux est constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés.

Le paramètre de solubilité global δ selon l'espace de solubilité de HANSEN est défini dans l'article "Solubility parameter values" de Grulke, dans l'ouvrage "Polymer Handbook" 3ème édition, Chapitre VII, pages 519-559 par la relation :

$$\delta = ( d_D^2 + d_P^2 + d_H^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.).

La définition des solvants dans l'espace de solubilité tridimensionnel selon HANSEN est décrite dans l'article de HANSEN : "The three dimensionnal solubility parameters" J. Paint Technol. 39, 105 (1967).

Parmi les composés liquides non aqueux ayant un paramètre de solubilité global selon l'espace de solubilité de HANSEN inférieur ou égal à 20 $(MPa)^{1/2}$, on peut citer les corps gras liquides, notamment les huiles, qui peuvent être choisies parmi les huiles naturelles ou synthétiques, carbonées, hydrocarbonées, fluorées et/ou siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou

d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle.

On peut également citer les hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.

On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines; et les huiles siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

On peut également citer les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.

Parmi les composés non aqueux susceptibles d'être employés, on peut également citer les monoalcools ayant un paramètre de solubilité global selon l'espace de solubilité de HANSEN inférieur ou égal à 20 $(MPa)^{1/2}$, c'est-à-dire les monoalcools gras aliphatiques ayant au moins 6 atomes de carbone, notamment 6 à 32, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

De préférence, le milieux non aqueux comprend des huiles siliconées volatiles, notamment cycliques ou linéaires, telles que les cyclodiméthylsiloxanes et les diméthylsiloxanes linéaires, et/ou des esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

Le choix du milieu non aqueux peut être effectué aisément par l'homme du métier en fonction de la nature des monomères constituant le polymère et/ou de la nature du stabilisant.

La dispersion de polymère peut être fabriquée comme décrit dans le document EP-A-749747. D'une manière générale, la polymérisation peut être effectuée en dispersion, c'est-à-dire par précipitation du polymère au cours de formation, avec protection des particules formées à l'aide d'un stabilisant.

On peut donc préparer un mélange comprenant les monomères initiaux ainsi qu'un amorceur radicalaire. Ce mélange est dissous dans un solvant de synthèse.

Les monomères sont solubles dans le milieu réactionnel, alors que le polymère n'y est pas soluble. Au fur et à mesure de la polymérisation, le polymère va précipiter et se trouver stabilisé par le stabilisant présent. On obtient ainsi des particules de polymères protégées en surface par le stabilisant.

On peut directement effectuer la polymérisation dans le milieu non aqueux qui peut donc jouer également le rôle de solvant de synthèse.

On peut également effectuer la polymérisation dans un solvant de synthèse, puis effectuer ensuite un échange de solvant, en remplaçant le solvant de synthèse par le milieu non aqueux.

Ainsi, lorsque le milieu non aqueux choisi est une huile non volatile, hydrocarbonée ou siliconée, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc de préférence un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque le milieu non aqueux choisi est une huile volatile, hydrocarbonée ou siliconée, on peut directement effectuer la polymérisation dans ladite huile qui joue donc également le rôle de solvant de synthèse. Les monomères doivent de préférence également y être solubles, ainsi que l'amorceur radicalaire, et le polymère obtenu doit y est insoluble.

Les monomères sont de préférence présents dans le solvant de synthèse, avant polymérisation, à raison de 5-80% en poids. La totalité des monomères peut être présente dans le solvant avant le début de la réaction, ou une partie des monomères peut être ajoutée au fur et à mesure de l'évolution de la réaction de polymérisation.

L'amorceur radicalaire peut être, par exemple, un composé azoïque ou peroxyde tels que l'azo-bis-isobutyronitrile ou le tertiobutylperoxy-2-éthyl hexanoate.

Les particules de polymère sont stabilisées en surface.

Dans un premier mode de réalisation, les particules peuvent être stabilisées en surface au fur et à mesure de la polymérisation, grâce à un stabilisant qui peut être notamment un polymère séquencé, un polymère greffé, et/ou un polymère

statistique, seul ou en mélange. La stabilisation peut être effectuée par tout moyen connu, et en particulier par polymérisation en présence du stabilisant.

De préférence, le stabilisant est présent dans le mélange au départ de la polymérisation. Toutefois, il est également possible de l'ajouter en continu, notamment lorsqu'on ajoute également des monomères en continu.

Dans un second mode de réalisation, le polymère peut être synthétisé dans un solvant de synthèse, puis mis en dispersion dans un milieu non aqueux de dispersion par ajout du dispersant, et le solvant de synthèse évaporé.

On peut utiliser 0,1 à 30% en poids de stabilisant par rapport au poids du mélange initial de monomères, et de préférence de 1 à 20% en poids, préférentiellement de 2 à 15% en poids, voire de 3 à 10% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant comportant une partie (séquences, greffons ou autre), présentant une certaine affinité pour le polymère formé lors de la polymérisation.

Lorsqu'on utilise un polymère statistique en tant que stabilisant, on le choisit de manière à ce qu'il possède une quantité suffisante de groupements le rendant soluble dans le solvant de synthèse envisagé.

Parmi les polymères greffés, on peut citer les polymères siliconés greffés avec une chaîne hydrocarbonée et les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également citer, comme polymère stabilisant :

- (a) les copolymères blocs greffés ou séquencés comprenant au moins un bloc de type polyorganosiloxane et au moins un bloc d'un polymère (i) issu de la polymérisation radicalaire ou (ii) issu d'une polycondensation, notamment de type polyéther, polyester ou polyamide, ou leur mélange, ledit copolymère pouvant comporter des entités fluorées;
  Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.
  Lorsque les copolymères blocs greffés ou séquencés comprennent au moins un bloc de type polyorganosiloxane et au moins un bloc polyéther, le bloc polyorganopolysiloxane peut être notamment un polydiméthylsiloxane ou bien encore un poly alkyl($C_2$-$C_{18}$) méthyl siloxane; le bloc polyéther peut être un poly alkylène en $C_2$-$C_{18}$, en particulier polyoxyéthylène et/ou polyoxypropylène. On peut ainsi utiliser les diméthicones copolyol ou encore les alkyl ($C_2$-$C_{18}$) méthicones copolyol, éventuellement réticulés. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".
  On peut aussi citer le lauryl diméthicone copolyol crosspolymer, par exemple le KSG31 ou KSG32 de Shin-Etsu, le cétyl diméthicone copolyol tel que le DMC 3071 de GE, et le diméthicone copolyol PPG-3-oléyl éther tel que le KF-6026 de Shin Etsu.

- (b) les copolymères blocs, greffés ou séquencés, de (méth)acrylates d'alkyle en C1-C4 et de (méth)acrylates d'alkyle en C8-C30. On peut citer le copolymère méthacrylate de stéaryle/méthacrylate de méthyle.

- (c) les copolymères blocs greffés ou séquencés comprenant au moins un bloc résultant de la polymérisation de monomère éthylénique, à une ou plusieurs liaisons éthyléniques éventuellement conjuguées, et/ou notamment de diènes; et au moins un bloc de polymère issu de la polymérisation radicalaire autre que diène, notamment issu de monomère vinylique, (meth)acrylique ou (meth)acrylamide, ou d'un polyéther, d'un polyester ou d'un polyamide, ou leurs mélanges.
  Notamment, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).
  Comme copolymères blocs greffés ou séquencés comprenant au moins un bloc résultant de la polymérisation d'au

moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthacrylate de méthyle) /polyisobutylène ou les copolymères greffés à squelette poly(méthacrylate de méthyle) et à greffons polyisobutylène.
Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.
On peut également utiliser comme stabilisant, des composés tels que :

- (d) les alkyl-dimethicones dans lesquels le groupement alkyl comprend 6 à 32 atomes de carbone, tels que le lauryle methicone et le stéaryle methicone, notamment Si tec LDM 3107 d'ISP, le cetyl dimethicone tel que l'ABIL Wax 9801, le behenoxydimethicone tel que l'ABIL 5440 de Goldschmidt

- (e) les dimethiconol ester de formule :

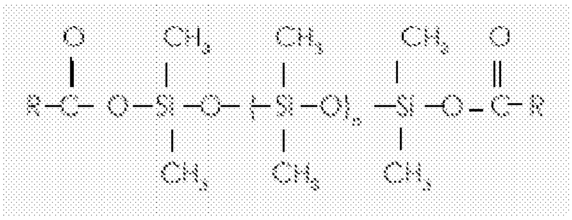

dans laquelle R est un radical alkyle ayant 6 à 32 atomes de carbone, tel que le dimethiconol behenate, et notamment les produits ULTRABEE de NOVEON et Pecosil DB de Phoenix Chemical.

- (f) les alkylamidoamines ayant notamment 6 à 60 atomes de carbone, notamment 12 à 50, telles que la behenamidopropyldimethylamine et notamment le Catemol 220 de Phoenix Chemical, de formule :

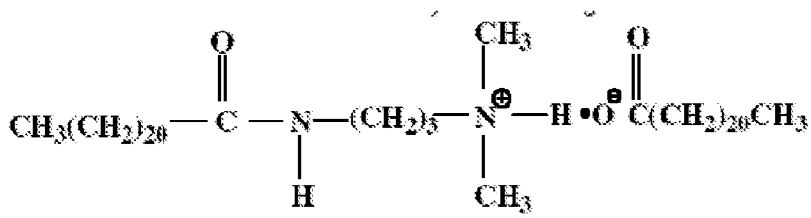

- (g) les copolymères comprenant au moins une partie polyorganosiloxane et des groupements fluorés, et notamment les silicones fluorées ou fluorosilicones qui peuvent être représentées par la formule :

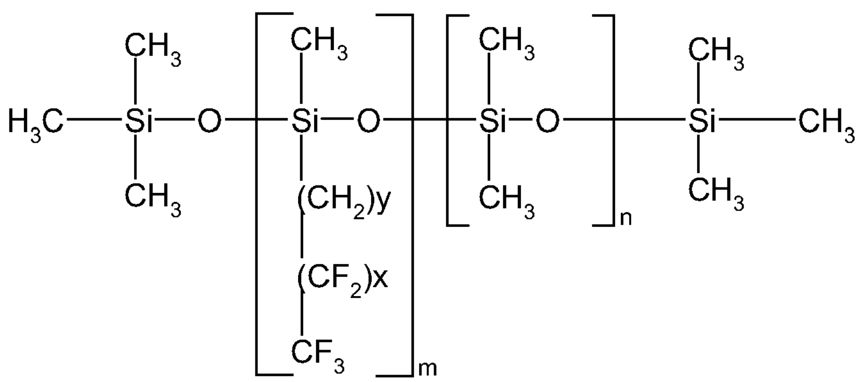

dans laquelle x est un entier compris entre 3 et 12, de préférence 5 et 10, notamment x=8; y est un entier compris entre 2 et 6, de préférence 2 ou 3; et m et n sont tels que le poids moléculaire du composé est compris entre 5000

et 15000;

et plus particulièrement les perfluorononyldimethicone, tels que ceux vendus sous la dénomination PECOSIL FSH-150 et 300 ou PECOSIL FSL-150 et 300 par Phoenix Chemical;

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.
Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.
Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

De manière particulièrement préférée, la dispersion selon l'invention est telle que :

- les monomères de Tg inférieure ou égale à -20°C, sont choisis parmi, seul ou en mélange, les acrylates d'éthyle, de propyle, de n-butyle, d'isobutyle, de 2-éthylhexyle, d'octyle, d'isooctyle, d'isodécyle, de décyle, de lauryle, de tridécyle, d'hydroxyéthyle et d'hydroxypropyle; les méthacrylates d'octyle, d'isooctyle, de décyle, d'isodécyle, de dodécyle, de lauryle, de tridécyle, de myristyle, de cétyle, de palmityle, de stéaryle, de béhényle, d'oléyle, et plus particulièrement comprend au moins de l'acrylate ou du méthacrylate d'éthyle 2-hexyle, et/ou
- les monomères de Tg inférieure ou égale à -20°C, représentent 50 à 100% en poids du poids total des monomères initiaux; et/ou
- l'agent stabilisant est choisi parmi les silicones fluorées ou fluorosilicones de formule :

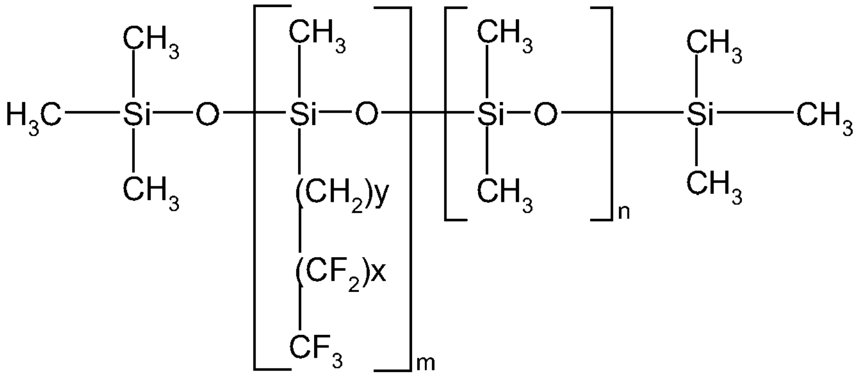

dans laquelle x=8, y = 2 ou 3, et m et n sont tels que le poids moléculaire du composé est compris entre 5000 et 15000; et/ou

- le composé liquide non aqueux est choisi parmi les huiles siliconées volatiles, notamment cycliques ou linéaires, telles que les cyclodiméthylsiloxanes et les diméthylsiloxanes linéaires, et/ou les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

Il est possible d'ajouter à la dispersion de polymères, un plastifiant de manière à abaisser la Tg des polymères utilisés. Le plastifiant peut être choisi parmi les plastifiants usuellement utilisés dans le domaine d'application et notamment parmi les composés susceptibles d'être des solvants du polymère. Le plastifiant peut être intégré lors de la synthèse ou ajouté une fois la synthèse réalisée.

Les dispersion obtenues selon l'invention peuvent alors être utilisées dans une composition notamment cosmétique ou pharmaceutique, qui comprend par ailleurs un milieu cosmétiquement ou pharmaceutiquement acceptable. Selon l'application envisagée, on peut utiliser des dispersion de polymères filmifiables ou non filmifiables, dans un milieu non aqueux comprenant des huiles volatiles ou non volatiles.

La composition peut alors comprendre, selon l'application envisagée, les constituants habituels à ce type de composition.

Parmi ces constituants, on peut citer les cires, les huiles, les gommes et/ou les corps gras pâteux, d'origine végétale, animale, minérale ou de synthèse, voire siliconés, et leurs mélanges.

Parmi les cires susceptibles d'être présentes dans la composition selon l'invention, on peut citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.

La composition selon l'invention peut également comprendre des huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée. Parmi les huiles susceptibles d'être présentes dans la composition selon l'invention, on peut citer, seules ou en mélange, les huiles hydrocarbonées telles que l'huile de paraffine ou de vaseline; le perhydrosqualène; l'huile d'arara; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

La composition selon l'invention peut également comprendre une ou plusieurs matières colorantes choisies parmi les composés pulvérulents et/ou les colorants liposolubles ou hydrosolubles, par exemple à raison de 0 à 70% du poids total de la composition et notamment de 0,01 à 70%.

Les composés pulvérulents peuvent être choisis parmi les pigments et/ou les nacres et/ou les charges habituellement utilisés dans les compositions cosmétiques ou pharmaceutiques. Avantageusement, les composés pulvérulents représentent de 0 à 50 % et notamment de 0,1 à 50 % du poids total de la composition et mieux de 1 à 40 %.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0 à 20 % et notamment 0,01 à 20 % du poids de la compositions et mieux de 0,1 à 6 %. Les colorants hydrosolubles sont par exemple le jus de betterave, le bleu de méthylène et peuvent représenter jusqu'à 6 % du poids total de la composition.

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des céramides, des filtres solaires, des tensioactifs, des polymères, des épaississants, des gélifiants. Bien entendu l'homme du métier veillera à choisir ce ou ces additifs et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous toute forme acceptable et usuelle pour une composition cosmétique ou pharmaceutique.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de

vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire.

Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques, des mousses ou des sprays. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

L'invention a encore pour objet un procédé de traitement cosmétique des matières kératiniques, notamment de la peau du corps ou du visage, des ongles, des cheveux et/ou des cils, comprenant l'application sur lesdites matières d'une composition cosmétique telle que définie précédemment.

Ce procédé selon l'invention permet notamment le maintien de la coiffure, le traitement, le soin ou le lavage ou le démaquillage de la peau, des cheveux ou de toute autre matière kératinique.

L'invention est illustrée plus en détail dans les exemples suivants, dans lesquels la Tg des polymères est calculée selon la loi de Fox; lorsque des monomères neutralisés sont employés, la Tg est alors "estimée", sur la base de la Tg de l'homopolymère obtenu à partir du monomère non neutralisé, étant donné que l'on sait que la neutralisation abaisse la Tg, lorsqu'elle est effectuée avec les neutralisants choisis.

## Exemples 1 à 46

On prépare des dispersions non aqueuses selon l'invention de la manière suivante: dans un réacteur, on introduit les composés ci-dessous mentionnés, sous agitation. On chauffe ensuite pendant 6 heures à 90°C.

| Composés (g) | Ex. 1 | Ex. 2 | Ex. 3 | Ex. 4 | Ex. 5 | Ex. 6 | Ex. 7 | Ex. 8 |
|---|---|---|---|---|---|---|---|---|
| acrylate de 2-ethyle hexyle Tg = -50°C | 2,4 | 2,4 | 2,4 | 2,4 | 2,4 | 2,4 | 2,4 | 2,4 |
| acide acrylique Tg = 105°C | 0,05 | 0,05 | 0,05 | 0,05 | 0,05 | 0,05 | 0,05 | 0,05 |
| Huile de silicone (D5) | 3 | 3 | 3 | 3 | 3 | 3 | 3 | 3 |
| Stabilisant 1 | 0,04 | 0,09 | | | | | | |
| Stabilisant 2 | | | 0,04 | 0,09 | | | | |
| Stabilisant 3 | | | | | 0,04 | 0,09 | | |
| Stabilisant 4 | | | | | | | 0,04 | 0,09 |
| Amorceur | 0,06 | 0,06 | 0,06 | 0,06 | 0,06 | 0,06 | 0,06 | 0,06 |

(suite)

| Composés (g) | Ex. 1 | Ex. 2 | Ex. 3 | Ex. 4 | Ex. 5 | Ex. 6 | Ex. 7 | Ex. 8 |
|---|---|---|---|---|---|---|---|---|
| Tg du polymère (calculée) | -48°C | -48°C | -48°C | -48°C | -48°C | -48°C | -48°C | -48°C |

| Composés (g) | Ex. 9 | Ex. 10 | Ex. 11 | Ex. 12 | Ex. 13 | Ex. 14 |
|---|---|---|---|---|---|---|
| acrylate de 2-ethyle hexyle | 2,4 | 2,4 | 2,4 | 2,4 | 2,4 | 2,4 |
| acide acrylique | 0,05 | 0,05 | 0,05 | 0,05 | 0,05 | 0,05 |
| Huile de silicone (D5) | 3 | 3 | 3 | 3 | 3 | 3 |
| Stabilisant 5 | 0,04 | 0,09 | | | | |
| Stabilisant 6 | | | 0,04 | 0,09 | | |
| Stabilisant 7 | | | | | 0,04 | 0,09 |
| Amorceur | 0,06 | 0,06 | 0,06 | 0,06 | 0,06 | 0,06 |
| Tg du polymère (calculée) | -48°C | -48°C | -48°C | -48°C | -48°C | -48°C |

| Composés (g) | Ex. 15 | Ex. 16 | Ex. 17 | Ex. 18 | Ex. 19 | Ex. 20 | Ex. 21 | Ex. 22 |
|---|---|---|---|---|---|---|---|---|
| acrylate de 2-ethyle hexyle | 2,4 | 2,4 | 2,4 | 2,4 | 2,4 | 2,4 | 2,4 | 2,4 |
| acide acrylique neutralisé AMP | 0,05 | 0,05 | 0,05 | 0,05 | 0,05 | 0,05 | 0,05 | 0,05 |
| Huile de silicone (D5) | 3 | 3 | 3 | 3 | 3 | 3 | 3 | 3 |
| Stabilisant 1 | 0,04 | 0,09 | | | | | | |
| Stabilisant 2 | | | 0,04 | 0,09 | | | | |
| Stabilisant 3 | | | | | 0,04 | 0,09 | | |
| Stabilisant 4 | | | | | | | 0,04 | 0,09 |
| Amorceur | 0,06 | 0,06 | 0,06 | 0,06 | 0,06 | 0,06 | 0,06 | 0,06 |
| Tg du polymère (estimée) | inférieure à -48°C | inférieure à -48°C | inférieure à -48°C | inférieure à -48°C | inférieure à -48°C | inférieure à -48°C | inférieure à -48°C | inférieure à -48°C |

| Composés (g) | Ex. 23 | Ex. 24 | Ex. 25 | Ex. 26 | Ex. 27 | Ex. 28 | Ex. 29 | Ex. 30 |
|---|---|---|---|---|---|---|---|---|
| acrylate de 2-ethyle hexyle | 2,4 | 2,4 | 2,4 | 2,4 | 2,4 | 2,4 | 2,4 | 2,4 |
| acide acrylique neutralisé AMP | 0,05 | 0,05 | 0,05 | 0,05 | | | | |
| Monomère SPE | | | | | 0,01 | 0,01 | 0,01 | 0,01 |
| Huile de silicone (D5) | 3 | 3 | 3 | 3 | 3 | 3 | 3 | 3 |
| Stabilisant 5 | 0,04 | 0,09 | | | 0,04 | 0,09 | | |
| Stabilisant 7 | | | 0,04 | 0,09 | | | 0,09 | 0,09 |
| Amorceur | 0,06 | 0,06 | 0,06 | 0,06 | 0,06 | 0,06 | 0,06 | 0,06 |
| Tg du polymère (estimée) | inférieure à -48°C | inférieure à -48°C | inférieure à -48°C | inférieure à -48°C | -- | -- | -- | -- |

| Composés (g) | Ex. 31 | Ex. 32 | Ex. 33 | Ex. 34 | Ex. 35 | Ex. 36 | Ex. 37 | Ex. 38 |
|---|---|---|---|---|---|---|---|---|
| acrylate de 2-ethyle hexyle | 2,4 | 2,4 | 2,4 | 2,4 | 2,4 | 2,4 | 2,4 | 2,4 |
| MADQUAT N-octyl | 0,01 | 0,01 | 0,01 | 0,01 | 0,01 | 0,01 | 0,01 | 0,01 |
| Huile de silicone (D5) | 3 | 3 | 3 | 3 | 3 | 3 | 3 | 3 |
| Stabilisant 1 | 0,04 | 0,09 | | | | | | |
| Stabilisant 2 | | | 0,04 | 0,09 | | | | |
| Stabilisant 5 | | | | | 0,04 | 0,09 | | |
| Stabilisant 7 | | | | | | | 0,04 | 0,09 |
| Amorceur | 0,06 | 0,06 | 0,06 | 0,06 | 0,06 | 0,06 | 0,06 | 0,06 |
| Tg du polymère (estimée) | inférieure à -48°C | inférieure à -48°C | inférieure à -48°C | inférieure à -48°C | inférieure à -48°C | inférieure à -48°C | inférieure à -48°C | inférieure à -48°C |

| Composés (g) | Ex. 39 | Ex. 40 | Ex. 41 | Ex. 42 | Ex. 43 | Ex. 44 | Ex. 45 | Ex. 46 |
|---|---|---|---|---|---|---|---|---|
| acrylate de 2-ethyle hexyle | 2,4 | 2,4 | 2,4 | 2,4 | 2,4 | 2,4 | 2,4 | 2,4 |
| MADAME neutralisé | 0,01 | 0,01 | 0,01 | 0,01 | 0,01 | 0,01 | 0,01 | 0,01 |
| Huile de silicone (D5) | 3 | 3 | 3 | 3 | 3 | 3 | 3 | 3 |
| Stabilisant 1 | 0,04 | 0,09 | | | | | | |
| Stabilisant 2 | | | 0,04 | 0,09 | | | | |
| Stabilisant 5 | | | | | 0,04 | 0,09 | | |
| Stabilisant 7 | | | | | | | 0,04 | 0,09 |
| Amorceur | 0,06 | 0,06 | 0,06 | 0,06 | 0,06 | 0,06 | 0,06 | 0,06 |
| Tg du polymère (estimée) | inférieure à -49°C | inférieure à -49°C | inférieure à -49°C | inférieure à -49°C | inférieure à -49°C | inférieure à -49°C | inférieure à -49°C | inférieure à -49°C |

- Stabilisant 1 : diméthicone copolyol (KF6017 de Shin Etsu)
- Stabilisant 2 : diméthicone copolyol (DC5225C de Dow Corning)
- Stabilisant 3 : acrylate/diméthicone copolyol (KP 545 de Shin Etsu)
- Stabilisant 4 : acrylates/stearyl acrylate/dimethicone acrylates copolymer (KP 561 de Shin Etsu)
- Stabilisant 5 : bloc polyoldiméthicone (Mw 8000) (GP675 de Genessee Polymer)
- Stabilisant 6 : mélange de cetyldimethicone copolyol, de poly-glyceryl isostearate (4 mol) et de laurate d'hexyle (ABIL WE -09 de Goldschmidt)
- Stabilisant 7 : perfluorononyl diméthicone (PECOSIL FSH-150 de Phoenix Chemical)
- Amorceur : tertiobutylperoxy-2-éthylhexanoate (Trigonox 21 S de Akzo).
- Monomère SPE : N,N-dimethyl-N-(2-methacryloyloxyethyl)-N-(3-sulfopropyl) ammonium bétaïne
- MADQUAT N-octyl : méthacrylate de N,N',N"-octyl diméthylammonium ethyl, de Tg inférieure à 20°C
- MADAME neutralisé : méthacrylate de diméthylaminoéthyle neutralisé à 100% par l'acide éthylcaproïque (le MADAME a une Tg = 19°C)

Dans tous ces exemples, on obtient au final une dispersion de particules de polymère, dans une huile de silicone volatile.

**Exemple 47**

Dans un réacteur de 1 litre muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min.
Une fois la température requise atteinte (90°C), on ajoute les constituants de la coulée, en 1 heure. On maintient la température à 90°C pendant 5 heures.

| | Pied de cuve (g) | Coulée (g) | Quantité totale (g) |
|---|---|---|---|
| Acrylate de 2-ethyle hexyle | 18 | 80 | 98 |
| Stabilisant 7 | 2 | -- | 2 |
| Huile de silicone (D5) | 90 | 90 | 180 |
| Amorceur (Trigonox 21 S) | 2 | 0.6 | 2.6 |

On obtient une dispersion stable de polyacrylate d'éthyl-2 hexyle dans une huile siliconée volatile (D5). La Tg calculée du polymère est de -50°C.
La mesure de la taille des particules, effectuée par diffusion quasi-élastique de la lumière avec un Coulter N4 SD, donne les résultats suivants :

- taille moyenne des particules : 150 nm
  Le poids moléculaire moyen en nombre, déterminé par GPC (solvant élution : THF) est de 19300 g/mol.
- polydispersité : 5.

**Exemple 48**

Dans un réacteur de 500 ml muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min.
Une fois la température requise atteinte (90°C), on ajoute les constituants de la coulée, en 1 heure et 20 minutes. On maintient la température à 90°C pendant 6 heures.

| | Pied de cuve (g) | Coulée (g) | Quantité totale (g) |
|---|---|---|---|
| Acrylate de 2-ethyle hexyle | -- | 70 | 70 |
| MADAME | -- | 30 | 30 |
| Acide éthylcaproïque | -- | 30 | 30 |
| Stabilisant 7 | 2 | 3 | 5 |

(suite)

| | Pied de cuve (g) | Coulée (g) | Quantité totale (g) |
|---|---|---|---|
| Myristate d'isopropyle | 50 | 150 | 200 |
| Amorceur (Trigonox 21 S) | 0,5 | 1 | 1,5 |

On obtient une dispersion stable de particules de polyacrylate d'éthyl-2 hexyle/MADAME neutralisé à 100%, dans le myristate d'isopropyle. La Tg estimée du polymère est inférieure à -33°C.

**Exemple 49**

Dans un réacteur de 500 ml muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min.
On note un blanchiment environ 30 minutes après que la température requise soit atteinte (90°C); on ajoute alors les constituants de la coulée, en 1 heure. On maintient la température à 90°C pendant 3 heures.

| | Pied de cuve (g) | Coulée (g) | Quantité totale (g) |
|---|---|---|---|
| Acrylate de méthyle (Tg = 10°C) | 24 | 6 | 30 |
| Acrylate de butyle (Tg = -54°C) | 14 | 56 | 70 |
| Stabilisant 2 | 5 | - | 5 |
| Huile de silicone (D5) | 215 | - | 215 |
| Amorceur (Trigonox 21 S) | 1 | 1,6 | 2,6 |

On obtient une dispersion stable de particules de polyacrylate de méthyle/acrylate de butyle, dans une huile de silicone. La Tg calculée du polymère est de -38°C.

**Exemple 50**

Dans un réacteur de 500 ml muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min.
On note un blanchiment environ 45 minutes après que la température requise soit atteinte (90°C); on ajoute alors les constituants de la coulée, en 50 minutes. On maintient la température à 90°C pendant 3 heures.

| | Pied de cuve (g) | Coulée (g) | Quantité totale (g) |
|---|---|---|---|
| Acrylate de méthyle (Tg = 10°C) | 24 | 6 | 30 |
| Acrylate de butyle (Tg = -54°C) | 14 | 56 | 70 |
| Stabilisant 7 | 15 | - | 15 |
| Huile de silicone (D5) | 215 | - | 215 |
| Amorceur (Trigonox 21 S) | 1 | 1,6 | 2,6 |

On obtient une dispersion stable de particules de polyacrylate de méthyle/acrylate de butyle, dans une huile de silicone. La Tg calculée du polymère est de -38°C.

**Exemple 51**

Dans un réacteur de 500 ml muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min.

On note un blanchiment environ 40 minutes après que la température requise soit atteinte (90°C); on ajoute alors les constituants de la coulée, en 1 heure et 10 minutes. On maintient la température à 90°C pendant 3 heures.

| | Pied de cuve (g) | Coulée (g) | Quantité totale (g) |
|---|---|---|---|
| Acrylate de méthyle (Tg=10°C) | 24 | 6 | 30 |
| Acrylate de butyle (Tg = -54°C) | 14 | 56 | 70 |
| Stabilisant 7 | 15 | - | 15 |
| Diméthacrylate de 1,3-butanediol | - | 3 | 3 |
| Huile de silicone (D5) | 215 | - | 215 |
| Amorceur (Trigonox 21 S) | 1 | 1,6 | 2,6 |

On obtient une dispersion stable de particules de polyacrylate de méthyle/acrylate de butyle réticulé, dans une huile de silicone. La Tg calculée du polymère est de - 38°C.

**Exemple 52**

Dans un réacteur de 500 ml muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min.
On note un blanchiment environ 30 minutes après que la température requise soit atteinte (90°C); on ajoute alors les constituants de la coulée, en 1 heure. On maintient la température à 90°C pendant 5 heures.

| | Pied de cuve (g) | Coulée (g) | Quantité totale (g) |
|---|---|---|---|
| Acrylate de 2-éthyl hexyle | - | 98 | 98 |
| Méthacrylate de dodecafluoroheptyle (Tg ≤ 50°C) | - | 2 | 2 |
| Stabilisant 7 | 5 | - | 5 |
| Huile de silicone (D5) | 200 | - | 200 |
| Amorceur (Trigonox 21S) | 1 | 1,6 | 2,6 |

On obtient une dispersion stable de particules de polyacrylate de 2-éthylhexyle/méthacrylate de dodécafluoroheptyle, dans une huile de silicone. La Tg estimée du polymère est inférieure à -48°C.

**Exemple 53**

On compare une composition comprenant les constituants suivants (les pourcentages sont exprimés en pourcentage en poids de matière active) :

| Constituant | Composition selon l'invention | Composition témoin | Composition comparative I | Composition comparative II |
|---|---|---|---|---|
| Dispersion de l'exemple 47 | 1,0 | - | - | - |
| Dispersion non aqueuse de polymère A | - | - | 1,0 | - |
| Solution non aqueuse de polymère B | - | - | | 1,0 |

(suite)

| Constituant | Composition selon l'invention | Composition témoin | Composition comparative I | Composition comparative II |
|---|---|---|---|---|
| Cyclopentasiloxane dimethicone copolyol | 0,5 | 0,5 | 0,5 | 0,5 |
| Cyclopentasiloxane D5 | 10,0 | 10,0 | 10,0 | 10,0 |
| Chlorure de triméthyl béhényl ammonium | 1,2 | 1,2 | 1,2 | 1,2 |
| Propylèneglycol | 2,5 | 2,5 | 2,5 | 2,5 |
| Conservateur | qs | qs. | qs. | qs. |
| Parfum | qs. | qs. | qs. | qs. |
| Acide citrique/soude | qs. pH 6,5 | qs. pH 6,5 | qs. pH 6,5 | qs. pH 6,5 |
| Eau | qsp. 100 | qsp. 100 | qsp.100 | qsp.100 |

Dispersion A : dispersion de copolymère acrylate de méthyle (90% en poids) et acide acrylique (10% en poids) à 25% dans la cyclopentadimethylsiloxane (D5). La dispersion est stabilisée par 2% de polymethylcetyl dimethyl methylsiloxane oxyéthylénée (ABIL EM 90 de GOLDSCHMIDT). Tg calculée du polymère : 17°C.

Solution B : solution de polyacrylate de 2-ethylhexyle à 50% dans la cyclopentadimethylsiloxane additionné après polymérisation de 2 % de polydimethylsiloxane à greffons perfluorononyle (PECOSIL FSH-150). Tg calculée du polymère : -50°C.

On applique 2 grammes de chaque composition sur des mèches de cheveux naturels caucasiens de 20 cm de longueur pesant 2,7 grammes. Les mèches sont malaxées, laissées poser 5 minutes puis rincées. Les mèches humides sont enroulées sur des bigoudis de 2 cm de diamètre, puis sont ensuite séchées au casque 30 minutes à 70°C. Après séchage, on évalue la tonicité de la boucle, le toucher des cheveux ainsi que le démêlage des mèches au peigne.

On note que les 3 formules apportent un niveau de tonicité à la boucle proche, et bien supérieur à celui obtenu avec la mèche témoin. Les qualités cosmétiques du toucher et le démêlage divergent fortement d'une mèche à l'autre. La mèche traitée par la composition selon l'invention possède un toucher proche de celui de la mèche traitée par le témoin. La mèche est douce, lisse, non grasse, et le démêlage est aisé.
Comparativement à la mèche traitée par la composition selon l'invention, la mèche traitée par la composition hors invention I possède un toucher rêche et le démêlage est difficile.
Comparativement à la mèche traitée par la composition selon l'invention, la mèche traitée par la composition hors invention II possède un toucher gras et collant.

En conclusion, seule la composition selon l'invention permet d'obtenir de bonnes propriétés cosmétiques de toucher et de démêlage, tout en conservant un bon niveau de coiffant.

## Revendications

1. Dispersion de particules d'au moins un polymère éthylénique stabilisé en surface par un agent stabilisant, dans un milieu non aqueux constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés; **caractérisée par le fait que** ledit polymère éthylénique présente une température de transition vitreuse (Tg) inférieure ou égale à -20°C.

2. Dispersion selon la revendication 1, dans laquelle le polymère éthylénique présente une température de transition vitreuse (Tg) comprise entre -150°C et -20°C, notamment entre -100°C et -25°C, préférentiellement entre -95°C et -30°C, voire entre -80°C et -35°C, et encore mieux entre -70°C et -40°C.

3. Dispersion selon l'une des revendications précédentes, se présentant sous forme de nanoparticules de polymère en dispersion dans le milieu non aqueux, lesdites nanoparticules ayant une taille comprise entre 5 et 600 nm,

notamment 10 à 500 nm, encore mieux 15 à 450 nm.

**4.** Dispersion selon l'une des revendications précédentes, dans laquelle le polymère est issu de la polymérisation d'un ou plusieurs monomères de Tg inférieure ou égale à -20°C, qui représente(nt) 100% en poids du poids total des monomères.

**5.** Dispersion selon l'une des revendications 1 à 3, dans laquelle le polymère est issu de la polymérisation d'un ou plusieurs monomères de Tg inférieure ou égale à -20°C, et d'un ou plusieurs monomères additionnels de Tg supérieure à -20°C.

**6.** Dispersion selon la revendication 5, dans laquelle le monomère additionnel, ou le mélange de tels monomères, est présent à raison de 0,01 à 50% en poids, par rapport au poids total des monomères, notamment de 0,1 à 40% en poids, voire de 1 à 30% en poids, ou encore de 5 à 15% en poids; et le monomère de Tg inférieure ou égale à -20°C, ou le mélange de tels monomères, est présent à raison de 50 à 99,99% en poids, notamment de 60 à 99,9% en poids, voire de 70 à 99% en poids, ou encore de 85 à 95% en poids, par rapport au poids total de monomères.

**7.** Dispersion selon l'une des revendications précédentes, dans laquelle le polymère éthylénique comprend 40% à 100% en poids, par rapport au poids total de monomères, notamment de 60 à 99% en poids, voire de 70 à 98% poids, et encore mieux de 60 à 95% en poids de monomère hydrophobe, seul ou en mélange.

**8.** Dispersion selon la revendication 7, dans laquelle le monomère hydrophobe a une Tg inférieure à -20°C.

**9.** Dispersion selon l'une des revendications 4 à 6, dans laquelle les monomères de Tg inférieure ou égale à -20°C, sont choisis parmi, seul ou en mélange :

- (i) les esters de l'acide acrylique de formule $CH_2$=CHCOOR1 avec R1 représentant (a) une chaîne carbonée, notamment hydrocarbonée (alkyle), ayant 2 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F), à l'exclusion de la chaîne tertiobutyle; ou bien R1 représentant (b) un groupement polyoxyéthylène comprenant de 5 à 30 motifs d'oxyde d'éthylène; ou encore R1 représentant (c) un groupe -R-($OC_2H_4$)n-H, avec R = alkyle en C1-C12 et n est un entier compris entre 5 et 30 inclus;
- (ii) les esters de l'acide méthacrylique de formule $CH_2$=C($CH_3$)COOR2 avec R2 représentant (a) une chaîne carbonée, notamment hydrocarbonée (alkyle), ayant 8 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi - OH et les atomes d'halogène (Cl, Br, I et F) ; ou bien R2 représentant (b) un groupement polyoxyéthylène comprenant de 5 à 30 motifs d'oxyde d'éthylène; ou encore R2 représentant (c) un groupe -R-($OC_2H_4$)n-H, avec R = alkyle en C1-C30 et n est un entier compris entre 5 et 30 inclus;
- (iii) les esters de vinyle de formule $CH_2$=CH-OCO-R3 avec R3 représentant une chaîne carbonée, notamment hydrocarbonée, ayant 2 à 12 atomes de carbone, linéaire ou ramifiée, saturée ou insaturée;
- (iv) les éthers de vinyle de formule $CH_2$=CHOR4 avec R4 représentant une chaîne carbonée, notamment hydrocarbonée, ayant 1 à 12 atomes de carbone, linéaire ou ramifiée, saturée ou insaturée;
- (v) les N-alkyl (méth)acrylamides de formule $CH_2$=CHCONR5R'5 ou $CH_2$=C($CH_3$)CONR5R'5 avec R5 et R'5 représentant indépendamment l'un de l'autre, un atome d'hydrogène ou une chaîne carbonée, notamment hydrocarbonée, ayant 6 à 28 atomes de carbone, linéaire, cyclique ou ramifiée, saturée ou insaturée, éventuellement aromatique (aryle, aralkyle ou alkylaryle), comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F) ; étant donné que l'un au moins des radicaux R5, R'5 est différent de l'hydrogène.

**10.** Dispersion selon la revendication 9, dans laquelle les monomères de Tg inférieure ou égale à -20°C, sont choisis parmi, seul ou en mélange :

- les acrylates d'éthyle, de propyle, de n-butyle, d'isobutyle, de 2-éthylhexyle, d'octyle, d'isooctyle, d'isodécyle, de décyle, de lauryle, de tridécyle, d'hydroxyéthyle et d'hydroxypropyle.
- les méthacrylates d'octyle, d'isooctyle, de décyle, d'isodécyle, de dodécyle, de lauryle, de tridécyle, de myristyle, de cétyle, de palmityle, de stéaryle, de béhényle, d'oléyle.

- le butyrate (ou butanoate) de vinyle, l'éthylhexanoate de vinyle, le néononanoate de vinyle et le néododécanoate de vinyle.
- l'éther de vinyle, le méthylvinyléther, l'éthylvinyléther, l'éthylhexylvinyléther et le butylvinyléther;
- le N-octylacrylamide et le N-octadécylacrylamide.

**11.** Dispersion selon la revendication 9, dans laquelle les monomères de Tg inférieure ou égale à -20°C, sont choisis parmi les acrylates de n-butyle, d'isobutyle, de 2-éthylhexyle, d'octyle, d'isooctyle, d'isodécyle, de décyle, de lauryle; ainsi que les méthacrylates d'octyle, d'isooctyle, de décyle, d'isodécyle, de dodécyle, de lauryle, de tridécyle, de cétyle, de palmityle, de stéaryle, d'oléyle; et leurs mélanges.

**12.** Dispersion selon l'une des revendications 5 à 6 ou 9 à 11, dans laquelle les monomères additionnels sont choisis parmi, seul ou en mélange :

- (i) les composés vinyliques de formule $CH_2$=CHR6 dans laquelle R6 est

  - un groupe hydroxyle;
  - un groupe alkyle linéaire ou ramifié, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
  - un groupe cycloalkyle en $C_3$ à $C_8$ tel que cyclohexane,
  - un groupe aryle en $C_6$ à $C_{20}$ tel que phényle,
  - un groupe aralkyle en $C_7$ à $C_{30}$ (groupe alkyle en $C_1$ à $C_4$) tel que 2-phényléthyle ou benzyle,
  - un groupe hétérocyclique de 4 à 12 chaînons contenant un ou plusieurs hétéroatomes choisis parmi O, N, et S,
  - un groupe hétérocycloalkyle (alkyle de 1 à 4 carbones), tel que furfuryle, furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi -OH, les atomes d'halogène (Cl, Br, I et F).

- (ii) les acrylates de formule $CH_2$=CHCOOR7 dans laquelle R7 est un groupe tertiobutyle, un groupe cycloalkyle en C3 à C8; un groupe aryle en C6 à C20 ; un groupe aralkyle en C7 à C30 (groupe alkyle en C1 à C4); un groupe hétérocyclique de 4 à 12 chaînons contenant un ou plusieurs hétéroatomes choisis parmi O, N et S; un groupe hétérocycloalkyle (alkyl de C1 à C4) tel qu'un groupe furfuryle; lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi le groupe hydroxyle et les atomes d'halogène (Cl, Br, I et F).
- (iii) les méthacrylates de formule $CH_2$=C($CH_3$)COOR8 dans laquelle R8 est :

  - un groupe carboné, notamment hydrocarboné (alkyle) ayant 1 à 6 atomes de carbone, linéaire ou ramifié, tel qu'un groupe méthyle, éthyle, propyle ou isobutyle, ledit groupe alkyle pouvant en outre être éventuellement substitué par un ou plusieurs substituants choisis parmi OH et les atomes d'halogène (Cl, Br, I et F) ;
  - un groupe cycloalkyle en C3 à C8 ;
  - un groupe aryle en C6 à C20 ;
  - un groupe aralkyle en C7 à C30 (groupe alkyle en C1 à C4) ;
  - un groupe hétérocyclique de 4 à 12 chaînons contenant un ou plusieurs hétéroatomes choisis parmi O, N et S;
  - un groupe hétérocycloalkyle (alkyle en C1-C4), tel qu'un groupe furfuryle ;

lesdits groupes cycloalkyle, aryle, aralkyle, ou hétérocyclique ou hétérocycloalkyle pouvant être éventuellement substitué par un ou plusieurs substituants choisis parmi OH, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par

un ou plusieurs substituants choisis parmi les groupes hydroxyle et les atomes d'halogène (Cl, Br, I et F).
- (iv) les (méth)acrylamides de formule $CH_2$=CHCONR9R'9 ou $CH_2$=C($CH_3$)CONR9R'9 dans lesquelles R9 et R'9, identiques ou différents, représentent un atome d'hydrogène ou un groupe alkyle en C1-C5, linéaire ou ramifié, tel qu'un groupe n-butyle, t-butyle, isopropyle.

**13.** Dispersion selon la revendication 12, dans laquelle les monomères additionnels sont choisis parmi le vinylcyclohexane, le styrène et l'acétate de vinyle; les acrylates de tertiobutyle, de t-butylcyclohexyle, de t-butylbenzyle, de furfuryle et d'isobornyle; les méthacrylates de méthyle, d'éthyle, de propyle, de n-butyle, d'isobutyle, de t-butylcyclohexyle, de t-butylbenzyle, de méthoxyéthyle, de méthoxypropyle et d'isobornyle; le N-butyl(méth)acrylamide, le N-isopropyl(méth)acrylamide, le N,N-diméthyl(méth)acrylamide et le N,N-dibutyl(méth)acrylamide, et leurs mélanges.

**14.** Dispersion selon l'une des revendications précédentes, dans laquelle le polymère éthylénique a un poids moléculaire moyen en nombre (Mn) compris entre 2000 à 1 000 0000, notamment entre 3000 et 800 000, et encore mieux entre 4000 et 500 000.

**15.** Dispersion selon l'une des revendications précédentes, dans laquelle ledit composé liquide non aqueux ayant un paramètre de solubilité global selon l'espace de solubilité de HANSEN inférieur ou égal à 20 $(MPa)^{1/2}$, est choisi parmi, seul ou en mélange, les corps gras liquides, notamment les huiles naturelles ou synthétiques, carbonées, hydrocarbonées, fluorées et/ou siliconées, éventuellement ramifiées.

**16.** Dispersion selon l'une des revendications précédentes, dans laquelle ledit composé liquide non aqueux est choisi parmi :

- les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle.
- les hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.
- les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines; et les huiles siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.
- les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.
- les monoalcools gras aliphatiques ayant au moins 6, notamment 6 à 32, atomes de carbone, la chaîne hydrocarbonée ne comportant pas de groupement de substitution, tels que l'alcool oléique, le décanol, le dodécanol, l'octadécanol et l'alcool linoléique.

**17.** Dispersion selon l'une des revendications précédentes, dans laquelle ledit composé liquide non aqueux est choisi parmi les huiles siliconées volatiles, notamment cycliques ou linéaires, telles que les cyclodiméthylsiloxanes et les diméthylsiloxanes linéaires, et/ou des esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

**18.** Dispersion selon l'une des revendications précédentes, dans laquelle l'agent stabilisant est choisi parmi les polymères séquencés, les polymères greffés et/ou les polymères statistiques, seul ou en mélange.

**19.** Dispersion selon l'une des revendications précédentes, dans laquelle l'agent stabilisant est présent à raison de 0,1 à 30% en poids par rapport au poids du mélange initial de monomères, de préférence de 1 à 20% en poids, préférentiellement de 2 à 15% en poids, voire de 3 à 10% en poids.

**20.** Dispersion selon l'une des revendications précédentes, dans laquelle l'agent stabilisant est choisi parmi :

- les polymères siliconés greffés avec une chaîne hydrocarbonée et les polymères hydrocarbonés greffés avec une chaîne siliconée.
- les copolymères greffés ayant un squelette insoluble de type polyacrylique avec des greffons solubles de type acide poly (12-hydroxy stéarique).
- les copolymères blocs greffés ou séquencés comprenant au moins un bloc de type polyorganosiloxane et au moins un bloc d'un polymère (i) issu de la polymérisation radicalaire ou (ii) issu d'une polycondensation, notamment de type polyéther, polyester ou polyamide, ou leur mélange, ledit copolymère pouvant comporter des entités fluorées; et notamment les copolymères greffés de type acrylique/silicone;
- les copolymères blocs, greffés ou séquencés, de (méth)acrylates d'alkyle en C1-C4 et de (méth)acrylates d'alkyle en C8-C30; et notamment le copolymère méthacrylate de stéaryle/méthacrylate de méthyle.
- les copolymères blocs greffés ou séquencés comprenant au moins un bloc résultant de la polymérisation de monomère éthylénique, à une ou plusieurs liaisons éthyléniques éventuellement conjuguées, et/ou notamment de diènes; et au moins un bloc de polymère issu de la polymérisation radicalaire autre que diène, notamment issu de monomère vinylique, (meth)acrylique ou (meth)acrylamide, ou d'un polyéther, d'un polyester ou d'un polyamide, ou leurs mélanges.
- les alkyl-dimethicones dans lesquels le groupement alkyl comprend 6 à 32 atomes de carbone, tels que le lauryle methicone et le stéaryle methicone, le cetyl dimethicone, le behenoxydimethicone;
- les dimethiconol ester de formule :

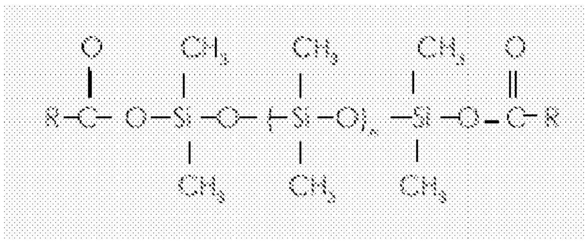

dans laquelle R est un radical alkyle ayant 6 à 32 atomes de carbone, tel que le dimethiconol behenate,
- les alkylamidoamines ayant notamment 6 à 60 atomes de carbone, notamment 12 à 50, telles que la behenamidopropyldimethylamine.
- les copolymères comprenant au moins une partie polyorganosiloxane et des groupements fluorés, et notamment les silicones fluorées ou fluorosilicones qui peuvent être représentées par la formule :

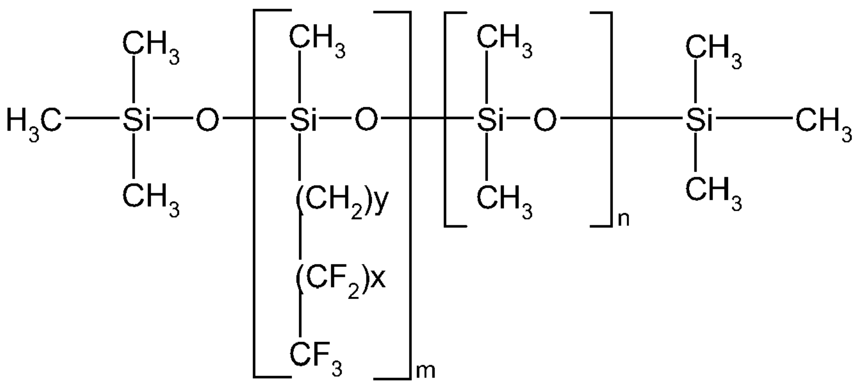

dans laquelle x est un entier compris entre 3 et 12, de préférence 5 et 10, notamment x=8; y est un entier compris entre 2 et 6, de préférence 2 ou 3; et m et n sont tels que le poids moléculaire du composé est compris entre 5000 et 15000;
- les copolymères blocs greffés ou séquencés comprenant au moins un bloc de type polyorganosiloxane et au moins un bloc polyéther, et notamment ceux pour lesquels le bloc polyorganopolysiloxane est un polydiméthylsiloxane ou un polyalkyl($C_2$-$C_{18}$) méthyl siloxane; le bloc polyéther est un polyalkylène en $C_2$-$C_{18}$, en particulier polyoxyéthylène et/ou polyoxypropylène.

- les diméthicones copolyol ou encore les alkyl ($C_2$-$C_{18}$) méthicones copolyol, éventuellement réticulés, tels que le lauryl méthicone copolyol; le lauryl diméthicone copolyol crosspolymer, le cétyl diméthicone copolyol et le diméthicone copolyol PPG-3-oléyl éther;
- les copolymères de type "dibloc" ou "tribloc" du type polystyrène/polyisoprène, polystyrène/polybutadiène, du type polystyrène/copoly(éthylène-propylène) ou encore du type polystyrène/copoly(éthylène-butylène);
- les copolymères bi- ou triséquencés poly(méthacrylate de méthyle)/polyisobutylène ou les copolymères greffés à squelette poly(méthacrylate de méthyle) et à greffons polyisobutylène.
- les copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, tels que les copolymères bi- ou triséquencés polyoxyéthylène/polybutadiène ou polyoxyéthylène/polyisobutylène.

**21.** Dispersion selon l'une des revendications précédentes, dans laquelle l'agent stabilisant est choisi parmi les silicones fluorées ou fluorosilicones de formule :

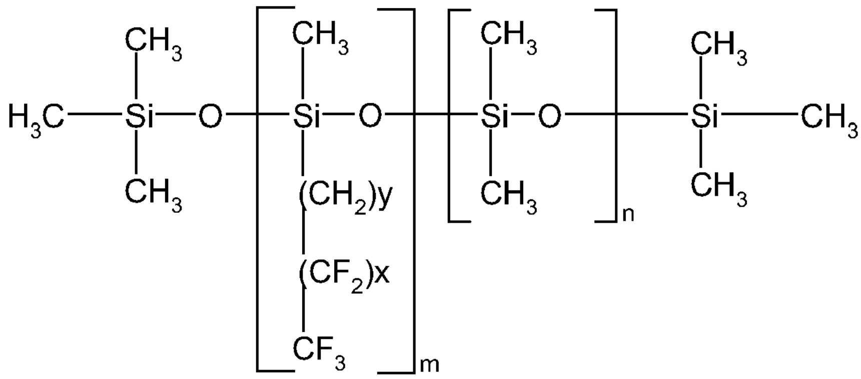

dans laquelle x=8, y = 2 ou 3, et m et n sont tels que le poids moléculaire du composé est compris entre 5000 et 15000.

**22.** Composition cosmétique ou pharmaceutique comprenant, dans un milieu cosmétiquement ou pharmaceutiquement acceptable, au moins une dispersion telle que définie selon l'une des revendications précédentes.

**23.** Composition selon la revendication 22, comprenant par ailleurs au moins un constituant choisi parmi les cires, les huiles, les gommes et/ou les corps gras pâteux, d'origine végétale, animale, minérale ou de synthèse, voire siliconés, et leurs mélanges; une ou plusieurs matières colorantes choisies parmi les composés pulvérulents et/ou les colorants liposolubles ou hydrosolubles; les antioxydants, les parfums, les huiles essentielles, les conservateurs, les actifs cosmétiques, les hydratants, les vitamines, les acides gras essentiels, les céramides, les filtres solaires, les tensioactifs, les polymères, les épaississants, les gélifiants.

**24.** Composition selon l'une des revendications 22 à 23, se présentant sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

**25.** Composition selon l'une des revendications 22 à 24, se présentant sous la forme d'un produit de soin et/ou de maquillage de la peau du corps ou du visage, des lèvres et des cheveux, d'un produit solaire ou autobronzant; d'un produit capillaire notamment pour le traitement, le lavage, le maintien ou la mise en forme des cheveux.

**26.** Composition selon l'une des revendications 22 à 25, se présentant sous la forme d'un shampooing, gel, lotion de mise en plis, lotion pour le brushing, composition de fixation et de coiffage telle que les laques, des mousses ou des sprays; d'un après-shampooing à rincer ou non, d'une composition pour permanente, défrisage, coloration ou décoloration, ou d'une compositions à rincer, à appliquer avant ou après une coloration, une décoloration, une permanente ou un défrisage ou encore entre les deux étapes d'une permanente ou d'un défrisage; de compositions lavantes pour la peau, et en particulier sous la forme de solutions ou de gels pour le bain ou la douche ou de produits

démaquillants; de lotions aqueuses ou hydroalcooliques pour le soin de la peau et/ou des cheveux.

**27.** Procédé de traitement cosmétique des matières kératiniques, notamment de la peau du corps ou du visage, des ongles, des cheveux et/ou des cils, comprenant l'application sur lesdites matières d'une composition cosmétique telle que définie à l'une des revendications 22 à 26.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1323753 A
- WO 9115185 A
- WO 9818433 A
- EP 749747 A


### Littérature non-brevet citée dans la description


- Polymer Handbook. John Wiley, 1989
- Solubility parameter values. **GRULKE.** Polymer Handbook. 519-559
- **HANSEN.** The three dimensionnal solubility parameters. *J. Paint Technol.,* 1967, vol. 39, 105